(19) Europäisches Patentamt
European Patent Office
Office européen des brevets

(11) Publication number: 0 208 647 A2

# (12) EUROPEAN PATENT APPLICATION

(21) Application number: 86630112.0

(22) Date of filing: 08.07.86

(51) Int.Cl.⁴: C 12 N 11/00
B 01 J 20/00, B 01 D 15/00
C 12 P 1/00

(30) Priority: 09.07.85 US 753286

(43) Date of publication of application:
14.01.87 Bulletin 87/3

(84) Designated Contracting States:
AT BE CH DE FR GB IT LI LU NL SE

(71) Applicant: PROTEIN FOODS GROUP INC.
1 West Avenue South
Hamilton Ontario L8N 2RN(CA)

(72) Inventor: Findlay, Christopher J.
R.R. No. 22, Cambridge
Ontario N3C 2V4(CA)

(74) Representative: Meyers, Ernest et al,
Office de Brevets Freylinger & Associés 46 rue du Cimetière B.P. 1153
L-1011 Luxembourg(LU)

## (54) Immobilisation supports for chemical and physical processes and methods of their manufacture.

(57) New immobilisation supports for use in physical and chemical processes are produced from bird, animal or fish bone by cleaning the bone of all tissue and finely dividing it to an appropriate particle size. Bone consists of a mineral matrix of calcium phosphate with organic connective collagen tissue material, principally the protein oseine, uniformly distributed therethrough. The oseine provides an ideal distributed site for the chemical attachment of bacteria, cells and organic enzyme catalysts, so that they are securely immobilised. It also constitutes a suitable medium, for example, for affinity chromatography in which complex molecules, such as antigens, form specific couples with support materials from which they can be washed out and decoupled for assay. The attachment may be by absorbtion, or by charge attraction, or may require a cross-linking agent attachable between the oseine and the supported material. The particular material to be attached may require the use of a chelating agent or a buffering agent to shield it from the action of the ionic calcium of the bone. A particularly economical source for the bone is the boney fraction obtained from the mechanical separation of meat and bone that is employed to recover meat from otherwise waste product, e.g. chicken necks and backs unwanted by the chicken fast food industry. Chicken bone is more porous than animal bone and thus particularly suitable for the purpose.

EP 0 208 647 A2

# IMMOBILISATION SUPPORTS FOR CHEMICAL AND PHYSICAL PROCESSES AND METHODS OF THEIR MANUFACTURE

## Field of the Invention

The present invention is concerned with new immobilisation supports for chemical and physical processes, and methods of making such new supports.

## Review of the Prior Art

Increasingly many modern chemical processes require the employment of immobilisation supports, for example, for the support of catalysts employed in a chemical reaction. An example of physical processes requiring a support are the various types of chromatography in which a column of support material selectively retains compounds from the fluid flowing through it, and subsequently the retained material is removed for assay. The effective support of catalysts has become increasingly important with the employment of catalysts which are cells or bacteria, or complex chemical substances, specific examples the latter being enzymes which are usually complex protein molecules. At least in commercial and pilot plant production, and advantageously in laboratory practice, a support must be provided for the catalyst which will maximize the surface available for catalytic activity, and will also retain the catalyst against physical removal by the flowing reacting materials, so as to provide for fast efficient reaction with minimum loss of the catalyst. Secure retention is usually needed to prevent undesirable contamination of the product with the catalyst. In the art this is referred to as immobilisation

of the catalyst on the support. Most important is the overall cost of immobilization which includes the effective cost of the support, the enzyme, and the immobilization procedure. To keep the cost as low as possible, it is desirable to implement inexpensive supports, crude enzyme preparations, and simple immobilizing schemes.

Typical supports that have been used hitherto are polymer gels (for example, alginates, agaroses and polyacrylamides) and cell wall material, but these are relatively soft materials that are not able to withstand high pressures and/or high flow rates of the chemical reactants over and/or through them without disruption and physical loss of the catalyst. Thus, if the rate of flow of the reacting materials is too high the catalyst is quickly exhausted by physical loss and continuous production is not possible, while if the rate is kept sufficiently low for this not to happen relatively large quantities of support and catalyst must be employed, with correspondingly large reactor vessels and high capital costs. It is of course possible to provide polymer gel supports of higher mechanical strength by increasing the internal polymer cross-linking so that more solid materials are obtained, but such materials are less pervious and also have a special problem of the potential toxicity of any residual monomer, or residual low molecular weight polymer, requiring expensive toxicological assessment before the particular batch can be employed.

Another product that has been used for catalyst support is glass beads of controlled pore size, the catalyst being immobilised thereon by charge attraction; it has been found that

this type of support has difficulty with fluid suspensions including particles larger than the pore size (e.g. fluid milk) with extensive fouling of the pores. Other inorganic supports include diatomaceous earth, silica, alumina and sand.

U.S. Patent No. 4,572,897 descloses carriers for immobilising enzymes in which carrier particles are prepared by binding inert filler particles with a water soluble binder and granulating the mixture. One of the very many inert fillers suggested is bone meal, although the preferred fillers are diatomaceous earth and cellulose fibre.

Definition of the Invention

In accordance with the present invention there is provided a new immobilisation support for use in chemical or physical processes consisting of cleaned, finely-divided bird or animal or fish bone.

Also in accordance with the invention there is provided a method of making an immobilisation support for chemical and physical processes including the steps of:-

a) cleaning any one or more of bird, animal and fish bone for the removal therefrom of tissue, and

b) finely dividing the cleaned bone.

The cleaned finely-divided bone may be provided with a cross-linking agent for the material to be retained on the support. The cross-linking agent is, for example, a bi-functional compound able to attach itself securely to the oseine of the bone and to the material to be retained. A suitable cross-linking agent for enzymatic catalyst support is, for example, glutaraldehyde which provides a reactive aldehyde

group for attachment of the supported enzyme.

Description of the Preferred Embodiments

Bone is a unique natural, mechanically-strong, abundant, non-toxic, composite, structural material, whether obtained from birds, animals or fish, consisting of a relatively inert mineral matrix of calcium phosphate, in the form of crystalline hydroxyapatite that is insoluble at physiological pH values, this matrix having a complementary matrix of a relatively stable organic connective collageneous tissue material, particularly the protein oseine, uniformly distributed therethrough. A protein-carbohydrate complex called the ground substance permeates the collagen/bone matrix and surrounds the collagenous fibres. A fourth major constituent is bone-inhabiting cells (osteocytes) each of which occupies its own cavity in the matrix. All bone is somewhat porous and these pores provide a large surface area upon removal of the various types of tissue with which it is associated. The bones of birds are much less dense and much more porous than those of animals, and will provide a corresponding larger surface area. The bones of fish are more porous but more brittle than those of birds and land animals and correspondingly less satisfactory. Since the collagen is distributed uniformly throughout the bone it forms an excellent medium for the immobilisation on the bone of different types of catalysts, particularly catalyst-containing cellular material and proteinaceous catalysts such as enzymes, which can readily be attached by surface adsorbtion, charge attraction, or chemically by use of suitable cross-linking agents. Thus, for chemical attachment the high collagen content

yields a correspondingly relatively high level of residual amino acids, and particularly carboxyl groups, which facilitate the attachment. Since in this case the bonding is chemical the immobilisation is likely to be more stable than with surface adsorbtion or charge attraction, so that the catalyst is not readily removed by physical action of the reacting solutions. Moreover, the catalyst is more likely to have sterically available active sites, so that it is more active than when physically immobilised on an inert support, such as a gel or glass beads.

The meat processing industries produce bone as a by-product much of which is not easily disposable. Much of the animal bone is cleaned of tissue and then ground to bone meal for use as animal feed or fertiliser. Another source is the poultry processing industry, especially for fast food restaurants, which has as a by-product large quantities of chicken necks and backs. The processing of these necks and backs for mechanical removal of as much as possible of the usable meat has become a standard practice and results in the production of separate meat and bony fractions, the latter consisting of about 60 - 70% by weight of finely-divided bone interspersed with unseparated meat. For example, in the meat and bone separation processes and apparatus described in U.S. Patent Serial No. 4,340,184 poultry parts are fed under pressure into a cylindrical chamber having a circumferential wall constituted by a porous screen of specific slot aperture size. The relatively soft tissue is expelled through the slot apertures while the harder bone and adhering meat is retained

and eventually discharged through a bony fraction outlet. Typically the meat will be of particle size less than about 850 microns, while the bone will be of particle size greater than about 850 microns.

The bony fraction thus obtained may be cleaned of tissue by washing each volume of bony fraction with two volumes of caustic soda of 1% concentration at 100°C, the mixture being suitably agitated for a period of about 8 minutes and the resulting extract of the soluble proteins removed for further processing to recover the useful protein and fat. Other periods and different concentrations can of course be used. For example, it is found that a fish bony fraction requires a wash of only 2 minutes duration at this temperature, and a longer wash will begin to dissolve the bone, while a lamb fraction requires a longer wash of about 15 minutes and a pork or beef fraction requires a still longer wash of about 20 minutes. Depending upon the specific composition of the bony fraction it may be subjected to a hot water wash prior to the digestion with caustic soda to remove and render fat that is readily removed by such simple treatment, so as to reduce the amount of alkali that is required. Again, if the alkali treatment alone does not effect sufficient removal of the unwanted tissue, it may be subjected to prior or post enzymatic treatment to hydrolyse the cellular tissue and render it more readily soluble when washed.

The treatment with strong alkali also has the advantage that it sterilises the resultant support material and renders it free of potential reproduction factors such as viruses, bacteria and cells, and also removes potential feed stocks for such

factors such as amino acids. Another advantage for some of the immobilization procedures is that the alkaline treatment leaves the support material positively surface charged, so that it is inherently ready to accept and immobilize a negatively charged supported component. The cleaned bone that is thereby obtained from this particular source is already finely-divided and suitable for use immediately as an immobilisation support. In many processes it will be preferred for the support to be of smaller particle size and the bone may be ground to the required size. Processes employing the support in the form of a fluidised bed will require the particle size to be in a specific uniform range, for example 1 to 2 mm, and this can be achieved by grinding and sieving. It may also be preferred for the bone to be of larger particle size, e.g. to pass through a 4 mesh screen, especially when it is required to fill a large reactor. The bone particles from the separation are frequently of about 0.5 cm size, and slivers of up to 4 cm length also occur. Other processes may of course produce finely divided bone of other size ranges and distribution. Bone is a natural, non-toxic, degradable material that is acceptable within quite wide limits as to particle size and volume content as a food constituent, so that it is more widely applicable to food processing systems. Thus, finely divided bone is already approved for use as a source of calcium in various food and vitamin supplements.

In a specific example, an assay of chicken bone fraction material from such a mechanical separator showed the following composition, expressed as approximate percentages by weight:

| | | | |
|---|---|---|---|
| Fat: | 8% | | |
| Protein: | 17% | (Collagen | 8%) |
| Bone: | 30% | (Calcium | 8%) |
| Water: | 45% | | |

One hundred (100) parts of the mixture was washed with 100 parts of water at 100°C and the fat centrifuged off, thereby removing almost all of the fat solids. The remaining liquid was added to another 100 parts of 2% sodium hydroxide solution (to obtain the required 1% concentration) and processed at 100°C for 8 minutes. The remaining solid material was removed by centrifuging and straining through a 40 mesh screen (sieve opening 0.42 mm) and assayed as:

| | | |
|---|---|---|
| Clean Bone | 66% | (calcium 32%) |
| Protein | 8% | |
| Water | 26% | |

The clean bone thus obtained was stored in a 25% brine solution for future use.

The liquid fraction obtained from the last-mentioned separation was neutralised with hydrochloric acid and drum dried to obtain about 18 parts of solid material of which 14 parts was protein and 4 parts salt. Alternatively, membrane dialysis could be used to obtain 14 parts of salt-free protein.

The solid chicken bone product that results is a coarse porous clean white irregular material, characterized as of plate form. The bone was originally finely-divided in the meat/bone separation process and with the final product it is found that about 40% by weight is retained on a 10 mesh screen (sieve opening 2 mm) while 60% is retained on a 40 mesh screen with

some finer particles in the 1 micron size being present; the product may therefore be characterized as being of size such that about 50% by weight is retained by a 20 mesh screen (sieve opening 0.84 mm). The pore size of different bone materials varies widely, as determined by examination with an electron scanning microscope of samples of fish (trout), beef, pork, lamb and chicken vertebrae.

The fish bone was found to be very much more porous than any of the other and at low magnification exhibited almost a "honeycomb" structure; the pores were generally large, ovoid in shape with the major axis transverse to the length of the vertebrae, and more uniform in size than in the other bones, varying in the photograph from about 50 to 250 microns along the major axes.

The chicken bone was less porous and at low magnification had the appearance of a somewhat porous piece of pumice stone; the high magnification showed pores of from about 100 to 225 microns.

The pork bone examined showed areas of large pores adjacent to areas of small pores, the large pores being from about 100 to 220 microns in size while the small pores are about 15 - 35 microns in size.

At low magnification the lamb bone had the external appearance of being very porous with pores of about 100 - 700 microns, but the respective high magnification photograph was of a surface that accidentally was a non-porous surface, so that more accurate measurement of pore size was not possible.

Finally, the beef bone examined in this manner showed

in the low magnification photograph a generally uniform but less porosity than the other bones, the section examined in the high magnification photograph being of highly irregular conformation with apertures from about 15 to 800 microns.

Proteinaceous catalyst materials and amino acids to be supported on the bone typically will have molecules of less than 1 micron size, while bacteria and yeasts will typically be of particle size in the range 1 - 5 microns. Clearly therefore these materials can lodge in the apertures in the bone with ready access by the ambient fluid, so that the surface available for attachment is increased enormously by this porosity.

It is a particular advantage of the supports of the invention that they are sterilised, without deactivation of the binding ability of the oseine, the treatment removing unwanted cellular material, bacteria and yeasts from the support, which materials may otherwise start their own fermentations, degradations, etc. Because of the stability of the bone it is also possible to pasteurise it, if necessary, for example by a heat treatment of about 65 - 75°C for a period of about 5 - 30 minutes.

This temperature stability of the support also gives the possibility of operating the catalysed process at elevated temperature, for example, at the maximum temperature for a proteinaceous enzymatic catalyst, without degradation of the support. All catalysed systems are temperature sensitive, and the reaction rate of enzymatic systems also increases with temperature up to the temperature ($T_D$) at which it begins to become denatured. Oseine is a stable protein which will

withstand a higher temperature than most enzymes. Moreover, the immobilisation increases the activation energy and renders the immobilised material more stable; there is therefore the possibility that the $T_D$ temperature can be shifted into a zone in which pasteurisation occurs and repasteurisation is not needed.

Some supported materials, particularly cellular materials, are attachable directly to the oseine of the support by adsorbtion, including charge attraction, or by entrapment in the porous material.

Direct adsorption to a solid support is one of the preferred methods of immobilization if feasible, because of its simplicity and low cost. Moreover, adsorption is a relatively easily reversible process which allows for ready recovery of the support after the catalyst has been exhausted and involves highly selective binding. In the case of cells this is via multipoint attachment which enables the cells to adhere to the support much more strongly than enzymes. Cell wall composition must be considered, including its charge, the age of the cell, and the ratio between the volume of the cell and its surface area. Additionally, properties of the support such as its composition, its surface charge, surface area and pore size play important roles. The actual charge on the support material limits the available choice of microorganisms for attachment as the adhesion phenomena is mainly based on electrostatic interactions between the charged microbial cells and the charged support. Since electrostatic interactions are involved adsorption will be affected by pH changes that occur as the

result of microbial metabolism. All cells that have been examined for attachment, including microorganisms, have a net negative charge. The charge of a cell is related to its surface ionogenic groups, which undergo dissociation according to the pH of the immediate environment, the ionization of carboxyl and amino groups according to Equations 1 and 2 below being apparently a critical reaction indicating a net positivity in highly acidic conditions and a net negativity in alkaline conditions.

$$-COOH \text{ ---------- } -COO^- + H^+ \qquad (1)$$

acid pH alkaline pH

$$-NH_3^+ \text{ ---------- } -NH_2 + H^+ \qquad (2)$$

acid pH alkaline pH

The pH values for optimum adsorption depend on the relative isoelectric points (iep) of the microbial cells. The strongest adsorption of most cells generally occurs at pH 3 - 6, and the majority of microorganisms studied have iep in the range of pH 2 - pH 3. For example, Leuconostoc mesenteroides has a iep of pH 3.0. At its iep the surface charge of a bacterium will be zero while if the pH of a bacterial suspension is above the iep of the carboxyl groups, ionizable hydrogens can be produced, which can conceivably be replaced by any other cation; the entire cell thus behaves as a large anion and is capable of combining with any cation. Alternatively, at pH values below the iep of the amino groups, the bacterial cell can assimilate additional

hydrogen ions; each cell will then exhibit a net positive surface charge and behave as a large cation. This charge reversal of some bacteria may not be observed except at extreme pH.

The advantage of using a porous support such as bone is related to the amount of surface area available because of this priority. The following is a list of the possible forces of attraction between microbial cells and the adsorbent surfaces:

1. chemical bonding (hydrogen, thio, amide and ester bonds)
2. ion pair formation ($-NH_3^+$....-OOC-)
3. ion triplet formation (-COO-....$Ca^{2+}$....-OOC-)
4. interparticle bridging (polyelectrolytes)
5. charge fluctuations
6. charge mosaics
7. charge attraction of opposite signs
8. electrostatic attraction between surfaces of similar charge (same net charge but different surface potentials)
9. electrostatic attraction due to image forces
10. surface tension
11. van der Waals forces of attraction
12. electromagnetic forces
13. hydrodynamic forces
14. diffusional forces
15. gravitational forces
16. positive chemotaxis (low cellular mobility)

Less numerous are the possible forces of repulsion:

1. charge repulsion between surfaces of similar charge
2. van der Waals forces of repulsion
3. steric exclusion (hindrance)
4. negative chemotaxis (high cellular mobility)

The time required to permit cell adsorption of the cellular material to the solid support must be considered and can be determined by monitoring the optical density of the cell suspension in the feed tank, with respect to time, during recirculation of the cell suspension over the solid support. Maximum cell loading can be considered as having been achieved when the optical density of the cell suspension reaches a constant minimum value, for example, for a period of at least two hours, the recirculation flow rate being maintained at a level that will not cause the cells to be washed off the support. In some procedures the cell suspension is recirculated for a determined amount of time followed by a period where the solution is allowed to stand in order to encourage maximum adsorption. The recirculating, as with agitation, increases the probability of contact between the microbial cells and adsorbent particles, but agitation can not be too long or too vigorous or it can cause desorption.

A carefully controlled drying procedure may be used to enhance adsorption by forcing a close contact between the cells and the support surface. Starving the cells (e.g. by immersion in pure water) may be used to promote adhesion. Starvation induces a modification of the cell wall and the release of ionic substances, thereby decreasing the electrostatic repulsion

between the cells and the support. However, a decrease, or loss of metabolic activity may be observed following such treatments. Although spontaneous adsorption is preferable it does not always occur, and to enhance the adsorption by decreasing repulsion or enhancing electrostatic attraction between cells and the supports, the support or the cell surface may be coated with a layer of positively charged colloidal particles such as Al $(OH)_3$ or $Fe_2O_3$, or metallic ions ($Fe^{3+}$, $Al^{3+}$).

Adsorbtion may also be used for the attachment of appropriate enzymes and other large-molecule chemical catalysts. Immobilized cells have several advantages over immobilized enzymes, in that it is not necessary to previously have extracted the enzyme from the cell. Furthermore, heat and operational stability in continuous enzyme reactions using intact cells are the same as, or superior to, those of immobilized enzymes. One disadvantage of using immobilized cells is that several different enzymes are usually in the cells and they may initiate side-reactions or degradation of the desired product. This can often be avoided by heat, acid and/or chemical treatment before or after immobilization of microbial cells.

Entrapment is based on the inclusion of the supported material within the rigid network constituted by the porous substrate to prevent its diffusion into the surrounding medium, while still allowing penetration of the substrate by the reacting fluid. Within this three dimensional network, the material is free in the compartments and pores.

Whereas the adsorption and entrapment methods are

particularly applicable to the immobilization of living cells, covalent coupling is more appropriately used with dead cells or cells to be utilized for only a single catalytic step. Covalent coupling methods have an advantage over the other methods by reducing or eliminating the problem of release or desorption of cells from the support, and while successfully used for enzyme immobilization, the attachment of whole cells to surfaces requires binding agents which generally are toxic toward the cells. Viable cells immobilized in this manner divide and form new unbound cells, resulting in substantial cell leakage The binding agents also represent an added cost.

Some catalytic agents may be attachable directly chemically to the oseine, but it is a relatively stable non-reactive protein and preferably is activated by use of a cross-linking agent, which will attach itself chemically to the oseine and provide a free bond for attachment of the catalyst. One such bi-functional cross-linking agent for use with enzymatic catalysts is glutaraldehyde which will provide a free aldehyde group for chemical attachment of the enzyme cell or biological reagent. In a typical procedure the cleaned finely-divided bone is immersed in an aqueous solution of the glutaraldehyde of concentration about 2% by volume for a period of about 10 minutes at a pH in the range 5.5 to 6.5. Concentrations of from 0.1% to 25% can be employed, and pH in the range from 3 to 10. The bone is then water washed two or three times to free it of excess glutaraldehyde and immersed in a solution of the required enzyme.

Other cross-linking agents that have been employed are:

Cyanogen bromide,

Hydrazine,

Carbodiimide,

and

Woodward's reagent K*

*N-ethyl-5-phenylisoxazolium-3-sulphonate sold by Sigma Biochemicals

Glutaraldehyde has the advantages of it's convenience in use, water solubility, ready availability and relatively very low toxicity.

The support of the invention has been employed for the support of the enzymes catalase; β-galactosidase (lactose); pectinases; porcine pepsin; glucose oxidase and glucose isomerase. It is found with some enzymes that account must be taken of the negative ionic effect of the calcium present in the crystalline portion of the bone; for example, pectinase will respond to the available calcium ion and gell, rendering it ineffective for enzymatic action. This effect can be reduced or avoided by "masking" the calcium, for example, by pretreatment with a calcium chelating agent, such as ethyldiaminetetraacetate (EDTA) or alginic acid; or a buffering agent such as sodium citrate and phosphates. The use of a buffering agent also provides the possibility of readily controlling the concentration of the enzyme on the support and thus it's specific activity, which can be adjusted to suit the application for which it is employed and perhaps avoid unnecessary provision of the costly material. In a specific example, the bone was treated with the buffer solution in the ratio of 10 mL of buffer

per gram of bone, and pectinase enzyme then applied in the concentration of 1 mg per mL of buffer; the resultant activated support showed activity of 10 mg of enzyme per 150 mg of oseine.

A series of tests were performed to evaluate different methods of attachment of well-known enzymes to chicken bone, the specific enzymes employed being:

*Candida* utilis invertase;

porcine stomach pepsin;

*Aspergillus* *niger* pectinase;

Lactozym (Trade Mark) 3000 L type HP (lactase) of Novo Industries; and

bovine liver catalase

The enzyme activity of the invertase was measured by reducing group evolution using 2-cyanoacetamide.

Milk clotting activity of the pepsin preparation was measured by timing the initial curd development of reconstituted skim milk (1:10 by volume) in 0.2M acetate buffer at pH 5.8, the activity being expressed as the reciprocal of clotting time in minutes (or milk clotting units) at 25°C.

Pectinase activity was measured as with the invertase. Lactase (β-galactosidase) activity was measured using o-nitrophenyl-D-galactopyranoside. The activity of the catalase was measured by the initial rate of oxygen evolution in the presence of 0.5 mM hydrogen peroxide in 0.5 M citrate-phosphate buffer at pH 5.0 using an oxygen polarograph.

Enzymatic activity on the bone is expressed in units per gram of dry bone; one unit of activity results in 1μ mol of substrate at 25°C being reacted per minute.

Adsorbtion without any pretreatment was achieved through the addition of the enzyme in an appropriate buffer to the clean dry bone followed by incubation under vacuum for 1 hour and 0°C. Excess enzyme was removed by exhaustive washing with buffer fluid prior to determination of the enzymatic activity. The same procedure for addition to the support was also employed after the respective pretreatment. The respective buffers used were:-

| | |
|---|---|
| Invertase: | 0.05 M acetate at pH 4.4 |
| Pepsin : | 0.5 M citrate at pH 4.2 |
| Pectinase: | As invertase |
| Lactase : | 0.2 M phosphate at pH 6.5 with 2 mM magnesium chloride at 5 mM cysteine |
| Catalase : | 0.1 M phosphate-citrate at pH 7.0 |

Acyl-azide cross-linking (derivitisation), glutaraldehyde derivitisation, with or without silanization, carbodiimide derivitisation and silanization were carried out as described elsewhere herein.

Treatment with collagenase to develop active sites prior to the addition of glutaraldehyde was carried out by incubating the bone in 0.2% (w/v) collagenase in 0.2 M phosphate buffer at 20°C (room temperature) for 8 hours.

The results of the tests are given in Table 1 below:

TABLE 1

| Enzyme | Immobilization Method | Initial Enzyme Activity in Immobilizing Solution U | Activity on Bone U/g |
|---|---|---|---|
| Invertase | Adsorption | 143 | 2.3 |
| | Acyl-Azide | 104 | 38 |
| | Glutaraldehyde (GHD) | 64 | 4.4 |
| | Carbodiimide | 28 | 1.0 |
| Pepsin | Adsorption | 43 | 0.68 |
| | Acyl-Azide | 38 | 0.63 |
| Pectinase | Acyl-Azide | 28 | 0.11 |
| | Glutaraldehyde (GHD) | 32 | 0.27 |
| | Carbodiimide | 30 | 0.10 |
| Lactase | Adsorption | 12 | 0.12 |
| | Acyl-Azide | 12 | 0.11 |
| | Glutaraldehyde (GHD) | 12 | 0.03 |
| | Silanized GHD | 24 | 0.10 |
| | Collagenase GHD | 24 | 0.16 |
| Catalase | Adsorption | 128 | 0.60 |
| | Acyl-Azide | 128 | 0.88 |

It will be seen that with the tests performed the most effective methods of attachment were adsorbtion and covalent coupling by acyl-azide. With catalase the acyl-azide system was superior to adsorbtion, while the reverse is true for lactose (ß-galactosidase). The uniformly superior results for invertase

will be noted, and also the uniformly good results for acyl-azide as compared to gluteraldehyde. The latter requires free amino groups which are not abundant in the collagenous oseine, so that the former using carboxylic acid and hydroxyl functional groups has more available sites. It will be noted however that with lactase the silanization pretreatment to develop free hydroxyl groups, and the collagenase pretreatment to liberate free amino groups, both raise the activity with glutaraldehyde to above that achieved with acyl-azide. Difficulty was experienced in evaluating the activity of the pectinase owing to gelling of the citrus pectin substrate, perhaps due to the high calcium level in the bone as described above, or contamination of the stock of enzyme.

The low yields of activity observed are believed to be indicative primarily of the relatively small number of active sites available, but the ready availability and economy of preparation of the granular bone material offers advantages over conventional support materials. The results of a study on flow characteristics of a granular bone column are given below. The application of the material to the support of pepsin has shown that in a sequential batch reactor system for the clotting of milk over 200 times the volume of the bed can be processed without loss of flow capacity or curd yield. Milk is a colloidal fluid food material which, because of its high content of large fat and protein molecules, chronically causes fouling of conventional support materials. An inexpensive securely-supported, high flow rate, long-life system cannot therefore be compared directly by these tests where the values

for initial enzyme activity would involve total loss of enzyme in a single batch reaction.

It is common in some known immobilisation systems to use filler material mixed with the support material, to improve flow characteristics and/or to extend the expensive support material, but this is not normally necessary with the support materials of the invention owing to their inherent structure and relatively low cost. It is unlikely therefore that the supported material used normally constitute more than 5 - 10% by weight of the total of support material plus supported material. For example, in the case of yeasts which are inherently large and bulky and also grow on the support, by the time that its weight has reached 5% of the total all of the cells would no longer adhere to the support, and the excess would no longer be immobilised. In the case of bacteria even if densities as high as $10^{10}$ per gram of support material could be achieved the total weight of the bacteria would still be less than 1% by weight of the total.

Another series of tests were carried out to compare the effectiveness of different immobilisation systems using some of the above cross-linking agents, as follows:-

Immobilization with the use of glutaraldehyde

1.0 g of chicken bone was suspended in 5 ml of 2 wt. % glutaraldehyde solution in a 0.1 M sodium phosphate buffer at pH 6.5. The mixture was kept under vacuum at room temperature for 30-60 min. The glutaraldehyde was removed and the treated bone was washed with distilled water. The bone was then treated with 5 m. of 0.05 M acetate buffer solution at pH 4.4, containing

varying amounts of polygalacturonase or invertase. Immobilization proceeded at standard conditions of 0-4°C for one hour under vacuum. The enzyme solution was then decanted and the bone washed thoroughly with acetate buffer (0.05 M, pH 4.4) and stored in the same buffer.

Immobilization using hydrazine

10 g of chicken bone was added to 15 ml of 0.05% (w/v) hydrazine sulphate solution in a 0.2 M sodium phosphate buffer at pH 7.0 and the suspension incubated at room temperature for 12 hours under vacuum. The hydrazine solution was then decanted and the bone thoroughly rinsed with 0.1 mM sodium chloride. The acyl-hydrazide bone was treated at 0°C with 10 ml of both 0.6 N HCl and 1 M sodium nitrite for 3-5 min. The nitrite was rinsed out with 250 ml of each of 0.1 M sodium chloride and 1 mM HCl. The bone was then resuspended in 15ml of solution of 0.05 M acetate buffer and pH 4.4, containing varying amounts of polygalacturonase or invertase. Enzyme coupling proceeded at standard conditions of 0°C for 3 hour under vacuum. The enzyme coupling solution was decanted and the bone thoroughly washed with acetate buffer (0.05 M, pH 4.4) and stored in the acetate buffer.

Immobilization with Woodward's Reagent K

300 mg of the Reagent K was added to a suspension of 1 g of bone in 5 ml of 0.1 M sodium phosphate buffer and pH 8.3. The reaction mixture was held under vacuum at room temperature for 1 hour. The solution of Reagent K was withdrawn and the bone rinsed thoroughly with distilled water. The treated bone was immersed with 5 ml of 0.05 M acetate buffer containing

varying concentrations of invertase. The mixture was magnetically stirred overnight at 4°C. The enzyme coupling solution was removed and the enzyme-treated bone was washed completely with acetate buffer (pH 4.4, 0.05 M), the bone being stored in 0.05 M acetate buffer at pH 4.4.

Immobilization using carbodiimide (cyanamide)

100 mg of cyanamide was added to a suspension of 1 g of bone as described above in 5 ml of 0.1 M sodium phosphate buffer of pH 7.0. The mixture was maintained under vacuum for 15-30 min. at room temperature. After extracting the carbodiimide solution, the bone was rinsed with distilled water. The treated bone was placed in 5 ml of 0.05 M sodium acetate buffer at pH 4.4 containing polygalacturonase or invertase at varying concentrations. Enzyme attachment proceeded at standard conditions of 0-4°C for 30 min. under vacuum. The enzyme solution was decanted and the bone washed thoroughly with the acetate buffer and stored in the same buffer.

Immobilization using cyanogen bromide

5 g of cyanogen bromide was added to a suspension of 25 g of bone in 200 ml of distilled water. While stirring, 1 M KOH was added dropwise to maintain the pH between 9.5-10.5. After 10 min. the cyanogen bromide solution was withdrawn and the bone washed with sodium bicarbonate at pH 8.0. The bone was immediately resuspended in 25 ml of 0.05 M acetate buffer at pH 4.4, containing polygalacturonase at concentrations used previously. Enzyme coupling proceeded at 0-4°C overnight under vacuum. When immobilization was completed, the treated bone was handled as before.

Enzyme Activity Assays

The activity of both polygalacturonase and invertase was assayed spectro-photometrically using the method of Gross (1982), a modification of the 2-cyanoacetamide procedure of Honda et al. (1982). The assay was based upon the enzyme-catalyzed hydrolytic release of reducing groups, galacturonic acid, and glucose and fructose by polygalacturonase and invertase respectively. Upon reacting 2-cyanoacetamide with reducing carbohydrates, ultraviolet-absorbing products were formed which could be determined.

Polygalacturonase activity was measured as follows. To 2.0 ml of 1% (w/v) polygalacturonic acid in 0.05 M acetate buffer (pH 5.0), samples of bone immobilized with enzyme, of decanted enzyme coupling solution or of soluble enzyme possessing polygalacturonase activity were added. The reaction proceeded while stirring for the given reaction period (1-10 min). The reaction mixture (2 ml) was poured into a large test tube containing 10 ml of 0.1 M borate buffer, pH 9.0, to which was added 2 ml of 1% (w/v) 2-cyanoacetamide. Samples were mixed and immersed in a boiling water bath for 10 min. After equilibriation to 25°C in an ice bath, the absorbance of the samples was read at 276 nm. The calibration curve was constructed using solutions of galacturonic acid containing 5-750 nm of galacturonic acid per volume of sample to be assayed. One unit of polygalacturonase was defined was that amount of enzyme required to liberate one micromole of galacturonic acid from the polygalacturonic acid solution at 25°C.

Invertase was determined similarly but with some modifications. To 5 ml of 0.05 M sucrose solution in 0.05 M acetate buffer, pH 5.0, a sample of bone immobilized with invertase, of decanted enzyme coupling solution, or of soluble enzyme possessing invertase activity was added. As the reaction progressed with agitation, aliquots of 0.4 ml were removed and added to a test tube that contained 2 ml of 0.1 M borate buffer, pH 9.0. After 0.4 ml of 1% (w/v) 2-cyanoacetamide was added, samples in test tubes were mixed and immersed in a boiling water bath for 10 min. After cooling to 25°C, the absorbance was measured at 276 nanometers. The calibration curve was constructed using equimolar solutions of glucose and fructose containing 5-1000 nanomols of each per 0.4 ml. One unit of invertase was defined as that amount of enzyme required to liberate one micromole of glucose in one minut for a sucrose solution at 25°C.

The following Tables 2 and 3 show the results of the different methods, and it is evident that glutaraldehyde was the best method of the techniques studied for immobilizing polygalacturonase, but the hydrazine technique was best of those examined for the attachment of invertase with the glutaraldehyde method producing lower but still favourable yields. No measurable activity was found on the bone treated with polygalacturonase using cyanogen bromide.

**TABLE 2**

**ACTIVITY OF IMMOBILIZED POLYGALACTURONASE USING DIFFERENT METHODS**

| Method * | Concentration of enzyme in coupling sol'n (mg/g dry bone) | Activity on bone (Units /g dry bone) | Yield as % of Soluble Activity ** |
|---|---|---|---|
| Glutaraldehyde | 11.07 | 0.266 | 1.10 |
| Hydrazine | 9.89 | 0.112 | 0.42 |
| Carbodiimide | 10.44 | 0.102 | 0.406 |
| Woodward's K | 8.02 | 0.096 | 0.436 |
| Cyanogen bromide | 8.50 | 0.0 | 0.0 |

**TABLE 3**

**ACTIVITY OF IMMOBILIZED INVERTASE USING DIFFERENT METHODS**

| Method * | Concentration of enzyme in coupling sol'n (mg/g dry bone) | Activity on bone (Units /g dry bone) | Yield as % of Soluble Activity ** |
|---|---|---|---|
| Glutaraldehyde | 0.40 | 4.43 | 7.04 |
| Hydrazine | 0.04 | 22.7 | 42.51 |
| Carbodiimide | 0.174 | 0.948 | 3.067 |

** Activity of polygalacturonase - 2.9 Units $mg^{-1}$ and activity of invertase - 160 Units $mg^{-1}$ (soluble).

The following Table 4 shows the effect of the pH of the coupling solution on the activity of immobilized invertase using hydrazine cross-linking agents.

TABLE 4

| Initial pH of Coupling | Final pH of Coupling | Activity (U/g dry bone) |
|---|---|---|
| 4.4 | 6.1 | 25.60 |
| 5.0 | 6.6 | 16.93 |
| 5.6 | 6.8 | 11.33 |

The following Table 5 shows the effect of the enzyme coupling time on the activity of immobilized invertase using hydrazine.

TABLE 5

| Coupling Agent | Time of Coupling (hr) | Activity (U/g dry bone) |
|---|---|---|
| Glutaraldehyde | 1.0 | 21.30 |
| | 2.0 | 21.02 |
| Hydrazine | 2.3 | 25.60 |
| | 3.0 | 23.95 |
| | 3.7 | 26.25 |

The following Table 6 shows the effect of invertase concentration in the coupling solution on the activity of immobilized invertase using hydrazine.

TABLE 6

| Concentration (mg/g dry bone) | Activity (U/g dry bone) | Yield (% of soluble activity) |
|---|---|---|
| 0.17 | 1.95 | 6.98 |
| 0.40 | 4.43 | 7.04 |
| 0.84 | 9.66 | 7.21 |
| 1.0 | 21.3 | 13.31 |
| 1.8 | 21.02 | 7.3 |

The effect of the pH of the coupling solution was that the activity of the immobilized invertase decreased as the pH of the enzyme coupling solution increased. This was likely the result of invertase inactivation caused by increases in the pH of the coupling solution during the immobilization as indicated in Table 4. The activity was not affected by coupling time as shown by the results in Table 5. This suggested that for a given enzyme concentration coupling took place immediately when the enzyme was introduced and possible saturation of enzyme coupling sites occurred within 1 and 2.3 hrs respectively using glutaraldehyde and hydrazine respectively. The activity was however affected by enzyme concentration in the coupling mixture. The results obtained reveal that activity of the immobilized invertase is directly related to the amount of available enzyme. It can be seen that approximately 7% of the enzyme was bound in most cases.

A further series of tests were carried out for the enzyme lactozym alone to compare the effectiveness of different methods of immobilization, as set out in Table 7 below. Cross

linking with glutaraldehyde was carried out both sequentially and simultaneously, and the sequential system was subjected to two different drying protocols, and employed also with silanization and pretreatment with collangenase to develop additional active sites.

In single stage drying the initially dry bone was incubated with 2% GHD in sodium phosphate buffer and pH 6.0 for 1 hour. The GHD-treated bone was washed exhaustively with distilled water, following which 5.0 ml of enzyme solution (2.4 units/ml) were added. The mixture was held at 0°C for 2.75 hours. After washing thoroughly with buffer the enzyme activity on the bone was determined as above. The procedure employed for two stage drying was as for single stage except that the washed, GHD-treated bone was oven-dried prior to the addition of the enzyme. The immobilization process involved the incubation of about 1 g of dry, GHD-treated bone with 12 units of enzyme activity for 1 hour at 0°C.

Silanizing prior to the GHD cross-linking was carried out by treating about 5 g of dry bone for 3 hours at room temperature with a 0.4% solution of 3-aminopropyltriethoxysilane ($\gamma$-APTES). The bone was then rinsed ten times with deionized water, and oven-dried. Immobilization involved the incubation of about 2 g of dry, silanized bone with 10 ml of 2% buffered GHD and pH 5.5). Following rinsing with sodium phosphate buffer at pH 5.5, 10 ml of enzyme solution (2.4 units/ml) were added, and the mixture was allowed to react at 0°C for 90 minutes. After thorough washing with buffer, the level of enzyme activity was determined.

The collagenase treatment involved the use of a buffered 0.2% solution at pH 7.0 of <u>Clostridium</u> <u>histolyticum</u> collagenase, the mixture being reacted overnight at 37°C. The bone was washed free of collagenase using distilled water, and oven-dried prior to treatment with GHD. Ten ml of enzyme solution (2.4 units/ml) were added to about 6 g wet, GHD-activated bone. Coupling proceeded for 90 minutes at 0°C. The enzyme-treated bone was then exhaustively washed with buffer solution.

Adsorption involved the incubation of about 2 g dry, untreated bone with 5.0 ml of enzyme solution (2.4 units/ml). Coupling proceeded for 1 to 2 hours at 0°C under vacuum.

The immobilization yields, in terms of units of enzyme immobilized per gram of support bone (absolute yield), were found to vary widely with the method of immobilization employed. The Table 7 below lists the immobilization methods used.

TABLE 7

| Method | Units/g Bone | % Yield |
|---|---|---|
| Adsorption | 0.124 | 9.9 |
| Hydrazine | 0.072 | 3.1 |
| GHD regular (sequential) | 0.019 | 0.4 |
| GHD - 2 point drying | 0.007 | 1.7 |
| GHD - 1 point drying | 0.035 | 1.0 |
| GHD + Silanization | 0.068 | 4.4 |
| GHD + Collagenase | 0.108 | 2.8 |
| GHD (simultaneous) | 0.005 | 0.1 |
| Woodward's Reagent K | 0.027 | 0.3 |
| CNBr Coupling | 0.012 | 0.1 |

As with the other tests the hydrazine and adsorption systems gave excellent results, with comparable results from the prior silanization and collagenase treatments.

The differences must be noted for the results of the regular (sequential) GHD treatment, and those obtained when similar experiments were conducted with the support being dried at particular points during the immobilization process, and these appear to show that reaction with the bone is hindered in the presence of moisture. Thus, the use of initially dry bone may allow for maximum exposure of the reactants to the bone surface. Furthermore, using dry bone, there is no dilution effect due to moisture at the surface, such a dilution effect would be quite pronounced due to the small sample sizes employed.

Another application of the material of the invention is in the field of affinity chromatography in which a fluid mixture to be assayed is passed through a column in which specific coupling reactions take place between constituents of the fluid and the material of the column. The usual prior art media for this procedure are various gells which are only capable of slow eleution. The porous bony material of the invention provides for rapid passage of the fluid through the column. When the required couples have been formed the column is washed to remove unwanted material. The wanted couples can then readily be uncoupled by rendering the support sufficiently acid, and washed out from the column. Such procedures are particularly suited for the separation of highly complex and delicate molecules such as antigens. With the supports of the invention it is possible to attach the required coupling agents to the oseine and because

of its stable and highly porous nature obtain much faster eleution times.

Flow characteristics

Tests were carried out to compare the pressure drop characteristic of unidirectional fluid flow through a packed bed of the chicken bone support material of invention, as compared with the drop through the same column of 'Dowex' ion-exchange resin (Lot No. MM-12141-Al) manufacturered by Dow Chemical Co., Midland, Michigan. The bed chamber consisted of 2.9 cm inside diameter pipe of methyl methacrylate resin, the total depth of the bed being 19.5 cm with a bed depth of 15.1 cm between upstream and downstream pressure measuring outlets. The pressure differential was measured using a mercury U-tube manometer, while the flow rates were measured by collecting the liquid that passed through the bed in a two litre cylinder, tap water being employed as the liquid and being fed to the packed bed at different flow rates.

The bed was packed so that settling was avoided during the tests, and to insure a constant porosity during the test run, an initial flow was maintained at the maximum operating pressure drop to compact the bed until no further change in porosity was detected. The bed was not disturbed until all flow tests were completed. The Reynolds number of the fluid was varied by varying the flow rate.

The pressure drops were measured to an accuracy of 0.5 mm of Hg, the data being corrected by subtracting the pressure drop in the empty bed and fittings. The equivalent particle diameter of the chicken bone was taken as the average of the

opening sizes of 10 and 20 mesh sieves, namely 1.246 mm, its bulk density being 495.6 kg/$m^3$. The corresponding equivalent particle size of the resin was 0.635 mm, while its bulk density was 462 kg/$m^2$.

Around Re=20, pressure drop through the ion exchange resin is 10 times the pressure drop through the crushed chicken bone.

Table 8 below tabulates the experimental data from which it will be seen that the chicken bone has much less pressure drop as compared to that through the ion exchange resin. For example at Re=19.3 for the bone the pressure drop is 21.2, while at Re=20.1 for resin the pressure drop is 224.3, an increase of more than 10 times. The value $\Delta P/V$ is found to be approximately linear for both the bone and the resin and the consistently higher value for the resin is apparent from the Table.

**TABLE 8**

**FLUID FLOW AND PRESSURE DROP DATA**

| Flow rate (V) $(l.m^{-2}.s^{-1})$ | Pressure drop( P) (kPa/m bed) | Reynolds No. (Re) | P<br>P/V |
|---|---|---|---|
| **Bone** | | | |
| 11.2 | 13.3 | 15.0 | 1.18 |
| 14.4 | 21.2 | 19.3 | 1.47 |
| 28.4 | 44.2 | 38.1 | 1.55 |
| 29.0 | 49.4 | 39.0 | 1.70 |
| 29.0 | 47.2 | 39.0 | 1.62 |
| 42.3 | 80.3 | 56.8 | 1.90 |
| 43.1 | 84.8 | 57.8 | 1.96 |
| 46.0 | 91.8 | 61.7 | 1.99 |
| 48.6 | 98.0 | 65.2 | 2.01 |
| 51.2 | 112.1 | 69.2 | 2.19 |
| **Resin** | | | |
| 11.6 | 106.0 | 7.9 | 9.1 |
| 19.1 | 186.3 | 13.1 | 9.75 |
| 22.2 | 191.6 | 15.2 | 8.63 |
| 26.2 | 204.0 | 17.9 | 7.78 |
| 29.3 | 224.3 | 20.1 | 7.65 |
| 33.8 | 260.5 | 23.1 | 7.70 |
| 34.7 | 281.7 | 23.7 | 8.11 |

Other Applications

The supports of the invention can of course be employed in any process in which the bone matrix is not appreciably degraded by the conditions of operation, and are particularly applicable to enzyme systems, since the support will usually have much greater tolerance of the operating conditions than will the enzyme itself. Owing to the by-product nature of poultry bone its cost is relatively low and the ease with which enzymes can be immobilised on the protein component renders it highly functional. Examples of suitable applications are:

a. The support of lactase enzyme for the continuous treatment of whey streams from cheese production to convert the lactose to galactose and glucose. A subsequent galactase enzyme would convert all the galactose to glucose which could be utilized as a support for the growth of yeast to produce alcohol or of lactic or acetic acid bacteria to produce food grade acidulants.

b. The support of glucose isomerase enzyme for the production of high fructose syrups as liquid sweeteners.

c. The support of pectinases for the clarification of fruit juices.

d. The support of proteases for the continuous "chill-proofing" of beer.

e. The support of specific proteases, like chymosin, for the continuous renneting of milk in the production of cheese.

f. The support of chicken pepsin on chicken bone support for use in continuous renetting in cheese production.

g. The support of glucose oxidase for the removal of glucose from egg white prior to freezing and drying.

h. The support of lipases for the production of specific fatty acids and interesterification of triglycerides.

i. The support of lactic acid bacteria, such as <u>Leuconostoc oenos</u>, in malolactic fermentation of wine for the fermentation conversion of L-malic acid to L-lactic acid and carbon dioxide. This is a secondary fermentation that usually occurs spontaneously after the alcoholic fermentation has been completed, and is required with wines from cooler climates to reduce acidity and provide stability after bottling. The necessary bacteria are present on the grape skins and perhaps also the processing vessels but with modern cleaner processing and requirement for reduced processing times there is no longer adequate time for the reaction to proceed, especially since the bacteria grow only slowly in the harsh conditions of wine with pH 3-3.8, alcohol content 10-14%, sulphur dioxide content of 10-100 ppm and low content of residual sugar. Inoculation is sometimes successful, but a safer approach is the use of a column reactor with the bacteria supported therein on a bone substrate of the invention. It is found that direct absorption is possible with a predicted effectiveness of 99% at 22.5°C for 42 minutes and pH 3.85. It maybe noted that the bone alone will reduce the malic acid content of the wine by 40 to 70%, it is believed by binding with the calcium in the bone.

j. The support of lactase, the lactose-splitting enzyme β-galactosidase, the action of which produces a milk product, such as fluid milk or whey, which possesses more desirable chemical and physical characteristics than its untreated counterpart. More specifically, low-lactose milk

would benefit persons who suffer from lactose intolerance (inability to digest lactose) and who normally refrain from consuming dairy products. Furthermore, lactose-hydrolyzed (LH) milk would be useful in the preparation of concentrated milk products where lactose crystallization causes textural problems. The development of a stable, immobilized lactase would also be of use in the treatment of whey and thereby aid in the abatement of the whey disposal problem currently faced by many cheese processing plants.

# CLAIMS

1. - A method of making an immobilisation support for chemical and physical processes characterised by the steps of:

a) cleaning any one or more of bird, animal and fish bone for the removal therefrom of tissue, and

b) finely dividing the cleaned bone.

2. - A method as claimed in claim 1, characterised in that the bone is finely-divided to be of particle size such that about 50% is retained by a 20 mesh sieve.

3. - A method as claimed in claim 1, characterised in that the bone is incorporated in a fluidized bed, the bone being of particle size of about 1-2 mm.

4. - A method as claimed in any one of claims 1 to 3, characterised by the step of attaching to the cleaned finely-divided bone a chemical cross-linking agent for the oseine and the material to be immobilised thereon.

5. - A method as claimed in claim 4, characterised in that the chemical cross-linking agent is selected from the group consisting of glutaraldehyde, cyanogen bromide, hydrazine, carbodiimide, Woodward's Reagent K.

6. - A method as claimed in any one of claims 1 to 5, characterised in that the bone is poultry bone.

7. - A method as claimed in any one of claims 1 to 6, characterised by the step of attaching to the cleaned finely-divided bone a catalytic material for immobilisation thereon.

8. - A method as claimed in claim 7, characterised in that the support material consists of not less than 95% by weight of the total of support and supported materials.

9. - A method as claimed in claim 7 or 8, characterised in that the material is an enzymatic catalytic material selected from the group consisting of bacteria, cells, vitamins, hormones and the enzymes catalase; β-galactosidase; pectinase; pepsin; lipase; glucose oxidase; glucose isomerase; galactase and protease.

10. - A method as claimed in any one of claims 1 to 9, characterised by the step of heating the support to a temperature of at least 65°C for a period sufficient to cause pasteurisation sterilisation thereof.

11. - A method as claimed in any one of claims 1 to 10, characterised by the step of treating the cleaned divided bone with a chelating agent for masking the ionic calcium thereof.

12. - A method as claimed in claim 11, characterised in that the chelating agent is EDTA.

13. - A method as claimed in any one of claims 1 to 10, characterised by the step of treating the cleaned divided bone with a buffering agent for masking the ionic calcium thereof.

14. - A method as claimed in claim 13, characterised in that the buffering agent is sodium citrate.

15. - A method as claimed in any one of claims 1 to 14, characterised in that the said cleaning step includes washing the bone with an alkaline solution capable of sterilisation thereof.

16. - A method as claimed in claim 15, characterised in that the bone is washed with a solution of caustic soda of about 1% concentration.

17. - A method as claimed in any one of claims 1 to 3 and 6 to 10, characterised in that the support is rendered alkaline to produce a positive charge thereon and the supported material is attached by charge attraction.

18. - A method as claimed in any one of claims 1 to 3 and 6 to 10, characterised in that the supported material is attached by adsorbtion.

19. - A method as claimed in any one of claims 1 to 3 and 6 to 10, characterised in that the surface of the support or the supported material is coated with a positively charged layer

to promote attachment.

20. - A method as claimed in any one of claims 1 to 19, characterised in that the support material is dried before attachment of the supported material thereto.

21. - A method as claimed in any one of claims 1 to 20, characterised in that the oseine of the bone is silanized to develop attachment sites thereon.

22. - A method as claimed in any one of claims 1 to 20, characterised in that the oseine of the bone is collagenized to develop attachment sites thereon.

23. - A method as claimed in any one of claims 1 to 22, characterised in that the supported material is incorporated in an absorbent column as the absorbing material.

24. - In a chemical process requiring the employment of a catalyst, the improvement characterised by the use as a support for the immobilisation of the catalyst of cleaned, finely-divided bird, animal or fish bone.

25. - In a physical process requiring the removal of a compound from a fluid by attachment to a supporting support, the improvement characterised by the use as a support of cleaned finely-divided bird, animal or fish bone.

26. - A new immobilising support for use in chemical or physical processes obtained by the method of any one of the claims 1 to 23.